# EUROPÄISCHE PATENTSCHRIFT

(11) **EP 0 764 172 B1**
(45) Veröffentlichungstag und Bekanntmachung des Hinweises auf die Patenterteilung: **01.09.2004**
(21) Anmeldenummer: 95920906.5
(22) Anmeldetag: 27.05.1995
(51) Int. Cl.: C07K 14/785, A61K 38/17

(54) **SYNTHETISCHE PEPTIDANALOGE DES LUNGENOBERFLÄCHENPROTEINS SP-C**
SYNTHETIC PEPTIDE ANALOGS OF LUNG SURFACTANT PROTEIN SP-C
ANALOGUES PEPTIDIQUES DE SYNTHESE DE LA PROTEINE SP-C DE SURFACTANT

(30) Priorität: 31.05.1994 DE 4418936
(43) Veröffentlichungstag der Anmeldung: 26.03.1997
(73) Patentinhaber: ALTANA Pharma AG, 78467 Konstanz (DE)
(72) Erfinder: NAVE, Rüdiger, D-78464 Konstanz (DE); ULRICH, Wolf-Rüdiger, D-78464 Konstanz (DE); STURM, Ernst, D-78465 Konstanz (DE); KRÜGER, Uwe, D-78464 Konstanz (DE); HÄFNER, Dietrich, D-78464 Konstanz (DE); SCHÄFER, Klaus, P., D-78465 Konstanz (DE); MELCHERS, Klaus, D-78267 Aach (DE)
(86) Internationale Anmeldenummer: PCT/EP1995/002028
(87) Internationale Veröffentlichungsnummer: WO 1995/032992

(56) Entgegenhaltungen:
- EP-A- 0 368 823
- WO-A-89/04326
- WO-A-91/18015
- DATABASE WPI Section Ch, Week 9344 Derwent Publications Ltd., London, GB; Class B04, AN 93-351660 & WO,A,93 21225 ( TOKYO TANABE CO) , 28.Oktober 1993

## Beschreibung

### Technisches Gebiet

Die Erfindung bezieht sich auf lungensurfactant-aktive Polypeptide, Verfahren zu deren Herstellung und diese enthaltende therapeutische Zusammensetzungen.

### Stand der Technik

Die Lunge aller Wirbeltiere enthält ein Substanzgemisch, das als "Lungensurfactant" bezeichnet wird. Es zeigt oberflächenaktive Eigenschaften und setzt die Oberflächenspannung im Alveolarbereich der Lungen so weit herab, daß ein Kollaps der finalen Atemwegsbereiche bei der Ausatmung vermieden wird. Dieses Substanzgemisch reguliert die Oberflächenspannung in einer dynamischen Art und Weise, so daß der nach dem Laplaceschen Gesetz zu erwartende Kollaps der kleinen Alveolen zugunsten der größeren durch entsprechende Anpassung der Oberflächenspannung vermieden wird. Als Ergebnis entsteht so eine wohl ausbalancierte, histologisch und physiologisch stabile Struktur der Lunge.

Lungensurfactant wird von den alveolären Pneumozyten vom Typ II in Form lamellarer Körperchen (lamellar bodies) sezerniert. Dieses sind kompakte Einheiten aus Phospholipid-Doppelschichten (bilayern) mit einem hohen Anteil an Dipalrnitoylphosphatidylcholin (DPPC) und Phosphatidylglycerin (PG). Als weitere essentielle Komponenten sind im Lungensurfactant Proteine enthalten, die mit SP-A, SP-B und SP-C bezeichnet werden. SP-A ist ein hochmolekulares Glycoprotein, das bei der Regulation der Sekretion eine entscheidende Rolle spielt.

Die Proteine SP-C und, in geringerem Maße, SP-B übernehmen bei der Ausbildung des monomolekularen Oberflächenfilms (dem Surfactant im engeren Sinne) die Rolle "thermodynamischer Katalysatoren". Durch die Anwesenheit dieser Proteine wird die Spreitungskinetik enorm beschleunigt. Erst dadurch ist die verzögerungsfreie Anpassung der Surfactant-Zusammensetzung an die jeweiligen Oberflächenspannungserfordernisse möglich. Diese Eigenschaften spiegeln sich in dem extrem hydrophoben Charakter der Proteine, insbesondere des SP-C, wieder.

Durch Extraktion von Lungengewebe oder Spülung von Tierlungen konnten Surfactant-Präparationen gewonnen werden, die sowohl in physikochemischen Meßapparaturen, wie in Tiermodellen, als auch bei klinischer Anwendung die Fähigkeit zeigen, einen Surfactant-Mangel auszugleichen und damit z. B. zur Therapie des kindlichen Atemnotsyndroms (IRDS) geeignet sind. Diesen Tierpräparaten sind jedoch gravierende Schwächen zu eigen:

Die Zusammensetzung der Phospholipide ist stark von Tierart, Gesundheit und Ernährungszustand des Tieres abhängig und kann nur begrenzt durch Zumischung definierter Komponenten ausgeglichen werden. Der Gehalt an Surfactant-Proteinen sowie das Verhältnis SP-B/SP-C ist den gleichen Unsicherheiten unterworfen. Hinzu kommt, daß eventuelle proteolytische Abbauprodukte der Proteine oder modifizierte Abkömmlinge (z. B. durch Oxidation am Methionin) ebenfalls in der therapeutisch eingesetzten Mischung enthalten sind. Bei einer längerfristigen Anwendung oder der Applikation großer Mengen an Surfactant, wie sie z. B. beim adulten Atemnotsyndrom (Schocklunge, ARDS) oder bei anderen Anwendungsfeldern, wie z. B. der Benutzung von Surfactant als "Schlepper" für andere Substanzen bei einer pulmonalen Applikation, nötig werden könnte, ist die Frage des Substanznachschubes offen.

Es bietet sich daher an, diese Probleme durch Herstellung der Proteine auf gentechnischem Wege zu lösen. Da rekombinante Proteine, insbesondere unter Verwendung bakterieller Expressionssysteme, in praktisch unbegrenzten Mengen herstellbar sind, und die Anwendung moderner Analysemethoden und Qualitätskontrollen möglich ist, kann unter Verwendung synthetischer Phospholipide ein Surfactant mit genau definierter Zusammensetzung hergestellt werden. Dieser kann an die therapeutischen Erfordernisse optimal angepaßt werden.

Das humane Protein SP-C (siehe Formel I, mit A = H oder Phe, B = Cys und C = Met), das für die Spreitungskinetik besonders wichtig ist, besteht in seinem zentralen Teil ausschließlich aus aliphatischen, sehr hydrophoben Aminosäuren, wie Valin, Leucin und Isoleucin. Die Länge dieses zentralen Teils (Aminosäuren 12-34) erlaubt die Integration des Peptids in den monomolekularen Phospholipidfilm. In der Sequenz Pro-Cys-Cys-Pro (Position 3-6) sind die beiden Cys-Reste durch Palmitinsäure an den SH-Gruppen thioverestert. Die Palmitinsäure erhöht den hydrophoben Charakter des Gesamtproteins weiter und verschließt gleichzeitig die beiden SH-Gruppen der Cysteine und schützt sie vor Oxidation und Disulfidbrückenbildung. Die zentrale Region (Aminosäuren 13-34) bildet eine Transmembran-Helix aus. Diese Region wird am N-Terminus flankiert durch eine polare Sequenz, die positiv geladene Aminosäuren (Lys, 10; Arg, 11) enthält.

In der WO 91/18015 wird die Herstellung von rekombinantem SP-C und von Mutanten des SP-C beschrieben. Es wird dort u. a. vorgeschlagen, die beiden Cysteine in Position 4 und 5 durch zwei Serine zu ersetzen. Dies hat bei der Herstellung den Vorteil, daß die nach der Isolierung des sehr hydrophoben Proteins technisch aufwendige Palmitoylierung der beiden Cysteine entfällt.

### Beschreibung der Erfindung

Es wurde nun überraschenderweise gefunden, daß SP-C-Mutanten, die sich von humanem SP-C durch Ersatz der beiden Cysteine in den Positionen 4 und 5 durch Phenylalanin oder Tryptophan und Ersatz des Methionin in Position 32 durch Isoleucin, Leucin oder Serin unterscheiden, keinerlei Funktionsverluste gegenüber dem natürlichen SP-C aufweisen und diesem bezüglich Stabilität sogar überlegen sind. Die gentechnologische Herstellung ist erheblich einfacher und ergibt höhere Ausbeuten. Die neuen Polypeptide mit Lungensurfactant-Aktivität lassen sich in sehr hoher Reinheit herstellen.

Gegenstand der Erfindung sind daher Polypeptide mit Lungensurfactant-Aktivität mit einer Aminosäuresequenz nach der allgemeinen Formel I, 
0 1 2 3 4 5 6 7 8 9 10
(A) Gly Ile Pro B B Pro Val His Leu Lys

11 12 13 14 15 16 17 18 19 20
Arg Leu Leu Ile Val Val Val Val Val Val (I),

21 22 23 24 25 26 27 28 29 30
Leu Ile Val Val Val Ile Val Gly Ala Leu

31 32 33 34
Leu C Gly Leu
 worin
A für H oder Phe,
B für Phe oder Trp und
C für Ile, Leu oder Ser stehen.

Ein bevorzugter Gegenstand der Erfindung sind Polypeptide der allgemeinen Formel I, worin A für H oder Phe, B für Phe und C für Ile stehen, wobei die Bedeutungen A = H, B = Phe und C = Ile besonders bevorzugt sind.

Ein weiterer Gegenstand der Erfindung sind pharmazeutische Zusammensetzungen, die durch einen Gehalt an einem oder mehreren erfindungsgemäßen Polypeptiden gekennzeichnet sind und gewünschtenfalls zusätzlich ein oder mehrere lungensurfactant-aktive Polypeptide aus der Gruppe SP-A und SP-B, vorzugsweise SP-B, enthalten.

Die erfindungsgemäßen Polypeptide können entweder durch die bekannten Methoden der Festphasen-Peptidsynthese oder mit Hilfe entsprechender rekombinanter Vektoren in Wirtszellen hergestellt werden. Die Techniken, Vektoren zu konstruieren, Zellen zu transformieren, die Exprimierung des Proteins in den transformierten Zellen zu bewirken und die exprimierten Proteine zu isolieren und zu reinigen, sind dem Fachmann an sich bekannt (z. B. WO 86/03408, WO 87/06588 und WO 91/18015).
Die Herstellung von Vektoren zur Exprimierung von SP-C in bakteriellen Systemen greift auf konventionelle Methoden der rekombinanten DNA-Technologie zurück.

Die Expression des hydrophoben SP-C-Proteins in Bakterien ist in größerer Menge und ohne Schaden für die Wirtszelle nur in Form geeigneter Fusionsproteine möglich wie z.B. zusammen mit der Chloramphenicolacetyltransferase (CAT). Z. B. kodiert der Vektor pTrpAmpCAT1 52 für die N-terminale Region von CAT und bietet für die "in frame"-Klonierung von DNA-Fragmenten, die für SP-C kodieren, eine (5'-)EcoR1- und eine (3'-)Pst1-Schnittstelle. CAT und SP-C werden hierbei auf Proteinebene über eine hydroxylaminsensitive Fusionsstelle (AsN↓Gly) miteinander verbunden. Solche Vektoren, wie pTrpAmpCAT152::SPC, erlauben die kontrollierte Expression der entsprechenden Fusionsproteine bis hin in den Produktionsmaßstab (Fermentation). Die Expression der Fusionsproteine bewirkt die Bildung von Inclusion Bodies in der Wirtszelle. Dabei kann die Länge des CAT-Anteils im Fusionsprotein so verändert werden, daß hohe Ausbeuten an Inclusion Bodies bei hohem SP-C-Anteil erzielt werden.

Die Exprimierung kann außer in Bakterien in einer Vielzahl von Wirtssystemen erfolgen, wie beispielsweise in Säuger-, Hefe- und Insektenzellen. Die für die verschiedenen Wirtszellen geeigneten DNA-Konstrukte werden nach den bekannten Methoden synthetisiert und auf übliche Weise mit den entsprechenden Steuersequenzen in das Genom der Wirtszellen eingebaut.

Zwei DNA-Oligonucleotide können nach der konventionellen Phosphoamidit-Methode auf einem MilliGen/Biosearch Cyclone DNA-Synthesizer synthetisiert werden.

Das erste DNA-Oligonucleotid bildet den sense-DNA-Strang mit einer Länge von 118 Nucleotiden. Dieser codiert (in 5'→3'-Richtung) für ein Eco R1-spezifisches 5'-Ende für spätere Subklonierung, die Asn/Gly Hydroxylamin-Spaltstelle und das humane SP-C beginnend mit dem Gly-25 und endend mit Leu-58 der SP-C-Vorläufersequenz, entsprechend Gly-1 bzw. Leu-34 in Formel I. Hierbei wurde die bekannte Aminosäure-Sequenz des humanen SP-C-Proteins nach den Regeln des genetischen Codes in DNA übersetzt. Die Sequenz wird jedoch in der Weise modifiziert, daß die beiden Cysteine in Position 28 und 29 der SP-C-Vorläufersequenz durch Phenylalanine oder durch Tryptophane und das Methionin in Position 56 der Vorläuferproteinsequenz durch Isoleucin, Leucin oder Serin ersetzt werden. Zusätzlich können auf diese Weise noch die Codonbenutzungshäufigkeiten der Wirtszellen berücksichtigt werden. Eine geänderte SP-C-Sequenz, die an den Positionen 4 und 5 Phenylalanin und in Position 32 Isoleucin enthält (Numerierung nach Formel I), wird entsprechend dem üblichen Einbuchstabencode für diese beiden Aminosäuren mit SPC34(FF/I) bezeichnet. Weiterhin enthält der sense-DNA-Strang ein TAA-Stoppcodon zur Termination der ribosomalen Translation sowie ein Pst 1-spezifisches 3'-Ende. Das zweite DNA-Oligonucleotid stellt den komplementären, nichtcodierenden (antisense) Strang bestehend aus 110 Nucleotiden dar.

Das synthetisch hergestellte SP-C-DNA-Fragment wird in einen geeigneten Expressionsvektor, wie z. B. pTrpAmpCAT152, einkloniert. Dieser Vektor setzt sich zusammen aus pKK233 (Pharmacia), der ein Ampicillin-Resistenz-Gen enthält, und ein Derivat von pBR322 ist. Der trc-Promotor kann durch einen trp-Promotor, wie in pTrpAmpCAT152, ersetzt werden. Andere induzierbare Promotoren sind ebenfalls einsetzbar.

Zur Subklonierung des SP-C-Fragments werden die komplementären DNA-Oligonucleotide zunächst miteinander hybridisiert. Der hieraus resultierende DNA-Doppelstrang weist überstehende Einzelstrang-Enden (Eco R1/Pst 1) auf.

Der Einbau in die Vektor-DNA erfolgt auf übliche Weise nach Eco R1/Pst 1-Verdauung der Vektor-DNA, Reinigung des gewünschten Vektor-DNA-Fragments durch Agarose-Gel-Elektrophorese und durch Hybridisierung der SP-C-DNA und des Vektorfragments über die köhäsiven Enden. Anschließend werden beide Fragmente nach bekannten Methoden durch Ligation miteinander kovalent verknüpft.

Zur DNA-Amplifikation und Plasmid-Isolierung wird nach gängigen Protokollen beispielsweise in Calciumchlorid-kompetente E.coli MM294-Zellen transformiert und zur Selektion von plasmidtragenden Zellen auf LB-Agarplatten mit Ampicillin plattiert. Aus den erhaltenen Amp-resistenten Kolonien wird Plasmid-DNA isoliert und diese mit geeigneten Restriktionsenzym-Kombinationen analysiert. Klone mit dem erwarteten DNA-Restriktionsfragmentmuster werden ausgewählt. Durch vollständige Sequenzierung der Plasmidsequenz wird die korrekt erfolgte Insertion der SPC34(FF/I)-Sequenz bestätigt.

Die so erhaltenen Plasmidvektoren erlauben die Expression des Fusionsproteins CAT::SPC unter Kontrolle des Trp-Promotors (oder anderer Promotoren). Das rekombinante Fusionsprotein fällt in den Wirtszellen nach Induktion in Form von Inclusion Bodies an.

Das Fusionsprotein CAT152::SPC34(FF/I) weist folgende, im üblichen Einbuchstabencode dargestellte Aminosäuresequenz auf:

10 20 30 40 50
MEKKI TGYTT VDISQ WHRKE HFEAF QSVAQ CTYNQ TVQLD ITAFL KTVKK
60 70 80 90 100
NKHKF YPAFI HILAR LMNAH PEFRM AMKDG ELVIW DSVHP CYTVF HEQTE
110 120 130 140 150
TFSSL WSEYH DDFRQ FLHIY SQDVA CYGEN LAYFP KGFIE NMFFV SANPE
160 170 180 186
FNGIP FFPVH LKRLL IVVVV VVLIV VVIVG ALLIG L
1 10 20 30 34

Die 34 Aminosäuren von SP-C(FF/I) in den Positionen 153 bis 186 des Fusionsproteins sind durch Unterstreichen und Angabe der Positionen 1 bis 34 gekennzeichnet.

Die spätere Spaltung mit Hydroxylamin zur Trennung von CAT und SP-C erfolgt zwischen Asn-152 und Gly-153 (entspricht der 1. Aminosäure im SP-C-Peptid). Die Abtrennung und Reinigung des SP-C-Peptids erfolgt nach den in der Proteinchemie üblichen Methoden.

Die erfindungsgemäßen Polypeptide können einzeln oder in Kombination miteinander in pharmazeutischen Zusammensetzungen zur Verfügung gestellt werden, die an die Erfordernisse der Atemwegsbehandlung angepaßt sind. Die Zusammensetzungen eignen sich nicht nur zur Behandlung des Atemnotsyndroms bei Frühgeborenen und Erwachsenen, sondern auch zur Behandlung von Pneumonien und Bronchitis. Außerdem eignen sich die erfindungsgemäßen Polypeptide als Schlepper für inhalativ verabreichbare Arzneistoffe.

Die Zusammensetzungen enthalten neben den Polypeptiden Phospholipide, vorzugsweise solche Phospholipide, die in natürlichen Lungensurfactant-Zusammensetzungen enthalten sind, wie vorzugsweise Dipalmitoylphosphatidylcholin (DPPC), Palmitoyloleylphosphatidylglycerol (POPG) und/oder Phosphatidylglycerol (PG). Zur Einstellung einer günstigen Viskosität enthalten die Zusammensetzungen Calcium- oder Magnesiumionen sowie Natriumchlorid. Der Fachmann orientiert sich bei der Bemessung der Art und Menge der einzelnen Bestandteile der Zusammensetzungen zum einen an der bekannten Zusammensetzung natürlicher Lungensurfactants und zum anderen an den zahlreichen Vorschlägen nach dem Stand der Technik, wie z. B. EP-A 0119056 und EP-A 0406732.

Bevorzugte erfindungsgemäße Zusammensetzungen enthalten 80 bis 95 Gew.-% Phospholipide, 0,5 bis 3,0 Gew.-% Polypeptide, 4 bis 7 Gew.-% Fettsäure, vorzugsweise Palmitinsäure, und 1 bis 3 Gew.-% Calciumchlorid.

### Herstellungsbeispiel

### 1. Produktionsstamm

Der verwendete Produktionsstamm E. coli 199 leitet sich von dem E. coli K12 Stamm MM294 ab, der unter der Nr. 5208 von der Deutschen Sammlung von Mikroorganismen und Zellkulturen GmbH (DSM, Braunschweig) bezogen werden kann.

Der das Gen des Fusionsproteins CAT152::SPC34(FF/I) enthaltende Expressionsvektor pTrpAmpCAT152::SPC34(FF/I) wurde aus der DNA-Sequenz pBR322, einem ColE1-Derivat [E. Weber (ed.) (1988), Biologische Sicherheit, Bundesministerium für Forschung und Technologie, Bonn], abgeleitet. In dem von Pharmacia erhältlichen Plasmid pKK233-2 wurde der Trc-Promotor mit Eco R1/Hind 3 herausgeschnitten und durch einen synthetischen Trp-Promotor ersetzt (pTrp233). Dieser besteht aus der Promotor-Region (Bindungsstelle für RNA-Polymerase), der Operator-Region (Bindungsstelle des Trp-Repressors), einer Shine/Da)garno-(S/D)-Sequenz sowie Restriktionsstellen zur Klonierung. Hinter den Trp-Promotor wurde das für die 152 Aminosäuren des 5'-Teils der bakteriellen Chloramphenicol-Acetyl-Transferase codierende Gen (CAT152) inseriert. An die CAT152-DNA-Teilsequenz wurde ein synthetisches Genfragment fusioniert, das für die 34 Aminosäuren des humanähnlichen SP-C(FF/I) kodiert. Die funktionelle CAT::SPC(FF/I)-Transkriptionseinheit wird abgeschlossen durch die bakterielle rrnB Transkripitons-Terminatorsequenz T1T2. Das erhaltene Konstrukt wird mit pTrpAmpCAT152::SPC34(FF/I) bezeichnet.

Der Vektor pTrpAmpCAT152::SPC34(FF/I) weist folgende Funktionselemente auf:
- CAT152::SPC34(FF/I)-Gen, gesteuert über Trp-Promotor und T1T2-Transkriptionsterminator;
- ori-Region und benachbarte Regionen, über die die Kopien-Zahl des Plasmids gesteuert wird;
- Amp®-Gen

Nach Einbringung des Plasmids in die Wirtszelle verfügt diese über eine hohe Kopienzahl des Trp-Promotor-kontrotlierten CAT::SPC(FF/I)-Gens. Der Trp-Repressor wird von der Wirtszelle selbst geliefert.

Über die Konzentration von Tryptophan im Medium bzw. über die Zugabe von β-IAA (β-Indolylacrylsäure) wird die fermentative Gewinnung von rCAT::SPC kontrolliert.

### 2. Batch-Fermentation

Mit einem Probenröhrchen der Working Cell Bank (Glycerinkultur) wird Kulturmedium (Zusammensetzung siehe unten) in einem Schüttelkolben beimpft (Vor- oder Starterkultur 1 l) und bei 37°C unter Schütteln unter starkem Ampicillin-Selektionsdruck inkubiert. Das Wachstum wird über die optische Dichte bei 578 nm verfolgt. Erreicht die E.coli 199-Starterkultur eine optische Dichte von mehr als 3, wird mit der Kultur ein 10 I-Fermenter beimpft und das Wachstum der Bakterien unter verringertem Ampicillin-Selektionsdruck fortgesetzt. Sobald die optische Dichte einen Wert zwischen 5 und 6 erreicht hat, wird die 10 I-Kultur in einen 100 I-Fermenter überführt und unter gleichen Bedingungen weiter inkubiert. Nach ausreichendem Wachstum wird durch Zugabe von beispielsweise 40 mg/l β-IAA die Trp-Promotor-gesteuerte CAT::SPC(FF/I)-Transkriptionseinheit induziert. Nach Induktion wird bis zur Zellernte für weitere 4-5 Stunden fermentiert.

Während der Fermentation werden Sauerstoffpartialdruck (pO₂), pH-Wert und Temperatur der Fermenterbrühe on-line erfaßt und reguliert. Der pH-Wert wird mit Natronlauge konstant gehalten, der Sauerstoffpartialdruck (pO₂) über Sauerstoffeintrag und Rührerdrehzahl geregelt. Off-line werden die optische Dichte bei 578 nm und die Konzentration der C-Quelle im Medium bestimmt. Schaumbildung wird mit einem Schaum-Sensor erfaßt, über den gegebenenfalls zur Schaumbekämpfung Antischaummittel zudosiert wird.

Aliquots der Kulturbrühe werden zu verschiedenen Zeitpunkten entnommen. Nach Lyse der Bakterien werden zur Kontrolle der Expression die E.coli-Proteine auf einem Polyacrylamid-Gel aufgetrennt und angefärbt. Der prozentuale Anteil der (dominanten) Proteine am E.coli-Gesamtprotein wird densitometrisch bestimmt. Kurz nach Induktion des rekombinanten Gens tritt eine neue dominante Proteinbande auf (rCAT::SPC).
Das Kulturmedium weist folgende Zusammensetzung auf:

Sojapepton 27,0 g/l, Hefeautolysat KAV 14,0 g/l, NaCl 5,0 g/l, K₂HPO₄ x 3 H₂O 6,0 g/l, KH₂PO₄ 3,0 g/l, MgSO₄ x 7 H₂O 0,5 g/l, Glycerin (99,5%ig) 30,0 g/l, Antischaum J673 (Struktol Comp.) 0,2 ml/l, L-Tryptophan 80,0 mg/l und Ampicillin 20 mg/l für die 1. Vorkultur und 5 mg/l für die 2. Vorkultur und den 100 l Fermenter.

Vor dem Autoklavieren und Sterilisieren des komplexen Nährmediums wird der pH-Wert mit 2N NaOH auf pH 6,8 eingestellt. Für die Vorkultur im 10 l Fermenter wird eine Rührgeschwindigkeit von 750 upm und ein Lufteintrag von 10 l/min bei 37°C und für die Hauptkultur im 100 l Fermenter eine Rührgeschwindigkeit von 400 upm und ein Lufteintrag von 70 l/min bei 37°C angewendet. Antischaummittel wird nach Bedarf mit einer Einmalspritze über ein Septum zugegeben.

Etwa 4 Stunden nach Induktion werden die Zellen durch Filtration und/oder Zentrifugation von der Kulturbrühe abgetrennt. Die feuchte Zellmasse wird in einem Edelstahlbehälter gesammelt und mit 10 l Aufschlußpuffer (pH 8,0) über Nacht im Kühlraum (16 Stunden, 4°C) gerührt. Die gerührte Zellsuspension wird im Hochdruckhomogenisator (≥700 bar) aufgeschlossen (Raumtemperatur) und erneut in einem sterilen Edelstahlbehälter gesammelt. Die Inclusion Bodies werden anschließend sofort durch Filtration und/oder Zentrifugation (Sorvallzentrifuge RC2-B, 27000 g) bei 4°C geerntet, in Puffer resuspendiert (ca. 1 l) und beispielsweise in Portionen von ca. 350 ml in 1 I-Rundkolben überführt und für etwa 96 Stunden lyophilisert. Aus einer 100 l Fermentation gewinnt man etwa 200 g trockene Inclusion Bodies mit einem Gehalt an Fusionsprotein von über 20 Gew.%. Die lyophilisierten Inclusion Bodies sind bei -20°C über Monate lagerbar.

### 3. Spaltung des Fusionsproteins und Reinigung des lipophilen Peptids SP-C(FF/I)

100 g trockene Inclusion Bodies werden in 1,6 l 8molarer Guanidin-Hydrochloridlösung (917,1 g) unter leichtem Erwärmen gelöst. Ungelöste Reste werden über ein Faltenfilter abfiltriert. Zur Spaltung des Fusionsproteins an der Asn-Gly-Verbindungsstelle werden der Lösung 167 g Hydroxylammoniumchlorid zugesetzt und der pH der Lösung mit 2N NaOH auf 9,6 eingestellt. Die Spalttösung wird dann für 3-4 Tage bei Raumtemperatur unter Rühren stehen gelassen. Am Ende der Reaktionszeit wird SP-C(FF/I) durch Zugabe von 6,4 l Tris-Puffer (pH 8,0) ausgefällt und mit Hilfe einer Zentrifuge (Sorvallzentrifuge RC2-B, 20000 g) niedergeschlagen. Der Überstand wird dekantiert, das SPC-Pellet in 400-500 ml Tris-Puffer erneut aufgeschwemmt und unter gleichen Bedingungen erneut für 30 Minuten zentrifugiert.

Das SP-C-Pellet wird in 3,5 l salzsaurem Chloroform/Methanol-Gemisch (1,75 l CHCl₃ + 1,75 l CH₃OH + etwa 30 ml 2N HCl) aufgenommen. Diese Roh-SP-C-Lösung wird durch präparative HPLC auf C8-Reverse Phase Material weiter gereinigt. Der Chloroform/Methanol-Extrakt wird vor dem Aufbringen auf die präparative HPLC Säule mit 90 %igem Methanol im Verhältnis etwa 1:2 verdünnt. Aus dieser Lösung läßt sich z. B. eine Säule (Durchmesser 5 cm) mit ca. 400 mg SP-C (FF/I) beladen (z. B. mit ∼2 l verdünntem Rohextrakt). Das SP-C (FF/I) wird unter sauren Bedingungen (pH 2-3) mit einem Wasser/i-Propanol-Gradienten eluiert (siehe Trennbedingungen). Nach etwa 30 Minuten Chromatographie werden im Bereich, in dem das SP-C eluiert (UV-Detektion bei 220 nm), 4-6 Fraktionen à 200 ml gesammelt. Die Fraktionen werden mit der analytischen HPLC überprüft und entsprechend gepoolt. Sollen die Proben gelagert werden, werden sie in flüssigem Stickstoff eingefroren und im Tiefkühlschrank bei -80°C aufbewahrt. Das SP-C(FF/I) wird in einer Reinheit von 98,5 - 99,5 % erhalten.

### Trennbedingungen:

| | |
|---|---|
| Säule | Kromasil C8 100 A 16µm 300 mm * 50mm I.D. |
| Eluent | A: HPLC-Wasser aus Millipore-Anlage |
| | B: i-PrOH gradient linear |
| | C: 60 mmol/l HCl (Verdünnung aus Salzsäure rauchend) |

### Gradient:

| Zeit | %A | %B | %C | Fluß [ml/min] |
|---|---|---|---|---|
| 0 | 45 | 50 | 5 | 100 |
| 10 | 45 | 50 | 5 | 100 |
| 55 | 0 | 95 | 5 | 100 |
| 65 | 0 | 95 | 5 | 100 |
| 75 | 45 | 50 | 5 | 100 |
| 85 | 45 | 50 | 5 | 100 |
| 90 | 45 | 50 | 5 | 0,2 |

### 4. Einbau von SP-C(FF/I) in eine Phospholipid-Matrix

Das lipophile Peptid SP-C(FF/I) wird in i-Propanollösung mit den Komponenten der Phospholipidmatrix versetzt und durch Einsprühen in eine verdünnte Kochsalzlösung (0,065 % w/w NaCl) bei Raumtemperatur in homogener Mischung mit den Komponenten der Phospholipidmatrix ausgefällt. Aus der Lungsurfactantsuspension wird der LSF mit einer Becherzentrifuge abgetrennt, in Elektrolytlösung (NaCl, CaCl₂) resuspendiert und der pH-Wert mit 0,1N NaOH auf pH 6,5 eingestellt. Diese wäßrige Suspension wird auf 20 ml Vials abgefüllt und lyaphilisiert. Die im folgenden Herstellungsbeispiel gemachten Gewichts- und Volumenangaben beziehen sich auf die Herstellung von 10 g Lungensurfactant-Präparation:

7,00 g Dipalmitoylphosphatidylcholin (DPPC), 3,08 g Palmitoyloleylphosphatidyl-glycerol-Ammoniumsalz (POPG x NH₄) und 0,25 g Palmitinsäure werden bei 40°C in 200 ml 90 % i-Propanol gelöst und dann auf Raumtemperatur abgekühlt. Die erhaltene Phospholipidlösung wird mit 1 l einer aus der HPLC-Reinigung erhaltenen, 200 mg gereinigtes SP-C(FF/I) enthaltenden Lösung vereinigt. Die erhaltene "Sprühtösung" wird unter Rühren mit Bikarbonatlösung (ca. 5 ml 5 % NaHCO₃-Lösung) auf pH 4,5 eingestellt.

Die "Sprühlösung" wird bei Raumtemperatur mit einer Einsprühgeschwindigkeit von 25 ml/min über eine Einstoffdüse in 9,6 l verdünnte NaCl-Lösung (0,065 % w/w) unter intensivem Rühren eingebracht. Es bildet sich eine opaleszierende Lösung, aus der nach zweistündiger Lagerung bei 4°-8°C die Lungensurfactant-Präparation durch Einsprühen einer Elektrolytlösung (3,0 g CaCl₂ x 2 H₂O und 61,3 g NaCl in 300 ml H₂O) ausgefällt wird. Die Lungsurfactant-Suspension (Gesamtvolumen 10,8-11,0 l) wird über Nacht bei 4°C gelagert und dann mit einer Sorvall-Becherzentrifuge (RC2-B) bei 16000 g in jeweils 30 Minuten abzentrifugiert. Der Zentrifugationskuchen wird jeweils zur Entfernung anhaftenden restlichen i-Propanols im halben Volumen 0,65 %iger Kochsalzlösung resuspendiert und erneut zentrifugiert. Diese Prozedur wird insgesamt 3-4 mal wiederholt. Der Kuchen der letzten Zentrifugation wird in 400 ml 0,65 %iger NaCl-Lösung aufgenommen, mit 0,1N NaOH auf pH 6,5 eingestellt und in Portionen zu 6,2 g auf 20 ml Vials aufgeteilt. Der Inhalt der Vials wird wie folgt lyophilisiert: Einfrieren über 6 Stunden bei -45°C und Normaldruck, Gefriertrocknen für 54 Stunden bei 0,16 mbar und -20°C, danach zur weiteren Intensivtrocknung noch 5 Stunden bei -20°C und 0,02 mbar.

Man erhält 65-66 Vials, die jeweils 0,150 g Lungensurfactant (ber. ohne NaCl) enthalten.

Die trockenen Lungensurfactant-Proben werden im Gefrierschrank bei 4°C gelagert und müssen vor dem Einsatz mit Wasser oder physiologischer Kochsalzlösung resuspendiert werden (Suspensionskonzentration 25 mg/ml).

Pro Vial sind enthalten:

| | |
|---|---|
| 95,6 mg | Dipalmitoylphospatidylcholin |
| 42,1 mg | Palmitoyloleylphosphatidylglycerol (Ammoniumsalz) |
| 2,7 mg | SP-C(FF/I) |
| 6,8 mg | Palmitinsäure |
| 2,9 mg | Calciumchlorid (wasserfrei) |

## Patentansprüche

1. Polypeptide mit Lungensurfactant-Aktivität der allgemeinen Formel I, 
0 1 2 3 4 5 6 7 8 9 10
(A) Gly Ile Pro B B Pro Val His Leu Lys

11 12 13 14 15 16 17 18 19 20
Arg Leu Leu Ile Val Val Val Val Val Val (I),

21 22 23 24 25 26 27 28 29 30
Leu Ile Val Val Val Ile Val Gly Ala Leu

31 32 33 34
Leu C Gly Leu
 worin
A für H oder Phe,
B für Phe oder Trp und
C für Ile, Leu oder Ser stehen.

2. Polypeptide nach Anspruch 1, **dadurch gekennzeichnet, daß**
A für H oder Phe,
B für Phe und
C für Ile stehen.

3. Polypeptid nach Anspruch 1, **dadurch gekennzeichnet, daß**
A für Phe,
B für Phe und
C für Ile stehen.

4. Polypeptid nach Anspruch 1, **dadurch gekennzeichnet, dass**
A für H,
B für Phe und
C für Ile stehen.

5. Pharmazeutische Zusammensetzung zur Behandlung des respiratorischen Distress-Syndroms (RDS) bei Säugern, **gekennzeichnet durch** den Gehalt an einem lungensurfactant-aktiven Polypeptid nach einem der Ansprüche 1 bis 4.

6. Pharmazeutische Zusammensetzung nach Anspruch 5, **dadurch gekennzeichnet, daß** mindestens ein weiteres lungensurfactant-aktives Polypeptid aus der Gruppe Lungensurfactant Protein SP-A und Lungensurfactant Protein SP-B enthalten ist.

7. Pharmazeutische Zusammensetzung nach Anspruch 6, **dadurch gekennzeichnet, daß** Lungensurfactant Protein SP-B enthalten ist.

8. Pharmazeutische Zusammensetzung nach einem der Ansprüche 5 und 6, **dadurch gekennzeichnet, daß** Phospholipide enthalten sind.

9. Pharmazeutische Zusammensetzung nach Anspruch 8, **dadurch gekennzeichnet, daß** Dipalmitoylphosphatidylcholin (DPPC), Palmitoyloleylphosphatidyl-glycerol (POPG) und/oder Phosphatidylglycerol (PG) enthalten sind.

10. Pharmazeutische Zusammensetzung nach Anspruch 8, **dadurch gekennzeichnet, daß** Palmitinsäure und Elektrolyte enthalten sind.

11. Pharmazeutische Zusammensetzung nach Anspruch 10, **dadurch gekennzeichnet, daß** als Elektrolyte Calcium- und/oder Natriumsalze enthalten sind.

## Claims

1. Polypeptides with pulmonary surfactant activity of the general formula I, 
0 1 2 3 4 5 6 7 8 9 10
(A) Gly Ile Pro B B Pro Val His Leu Lys

11 12 13 14 15 16 17 18 19 20
Arg Leu Leu Ile Val Val Val Val Val Val (I),

21 22 23 24 25 26 27 28 29 30
Leu Ile Val Val Val Ile Val Gly Ala Leu

31 32 33 34
Leu C Gly Leu
 wherein
A is H or Phe,
B is Phe or Trp and
C is Ile, Leu or Ser.

2. Polypeptides according to claim 1, **characterized in that**
A is H or Phe,
B is Phe and
C is Ile.

3. Polypeptide according to claim 1, **characterized in that**
A is Phe,
B is Phe and
C is Ile.

4. Polypeptide according to claim 1, **characterized in that**
A is H,
B is Phe and
C is Ile.

5. Pharmaceutical composition for the treatment of respiratory distress syndrome (RDS) in mammals **characterized by** an amount of a pulmonary surfactant-active polypeptide according to one of claims 1 to 4.

6. Pharmaceutical composition according to claim 5, **characterized in that** at least one further pulmonary surfactant-active polypeptide of the group of pulmonary surfactant protein SP-A and pulmonary surfactant protein SP- B is contained.

7. Pharmaceutical composition according to claim 6, **characterized in that** pulmonary surfactant protein SP-B is contained.

8. Pharmaceutical composition according to one of claims 5 and 6, **characterized in that** phospholipids are contained.

9. Pharmaceutical composition according to claim 8, **characterized in that** dipalmitoylphosphatidylcholine (DPPC), palmitoyloleylphosphatidylglycerol (POPG), and/or phosphaticylglycerol (PG) are contained.

10. Pharmaceutical composition according to claim 8, **characterized in that** palmitic acid and electrolytes are contained.

11. Pharmaceutical composition according to claim 10, **characterized in that** as electrolytes calcium salts and/or sodium salts are contained.

## Revendications

1. Polypeptides ayant une activité de surfactant pulmonaire de formule générale I : 
0 1 2 3 4 5 6 7 8 9 10
(A) Gly Ile Pro B B Pro Val His Leu Lys

11 12 13 14 15 16 17 18 19 20
Arg Leu Leu Ile Val Val Val Val Val Val (I),

21 22 23 24 25 26 27 28 29 30
Leu Ile Val Val Val Ile Val Gly Ala Leu

31 32 33 34
Leu C Gly Leu
 dans laquelle
A représente H ou Phe,
B représente Phe ou Trp et
C représente Ile, Leu ou Ser.

2. Polypeptides selon la revendication 1, **caractérisés en ce que**
A représente H ou Phe,
B représente Phe et
C représente Ile.

3. Polypeptide selon la revendication 1, **caractérisé en ce que**
A représente Phe,
B représente Phe et
C représente Ile.

4. Polypeptide selon la revendication 1, **caractérisé en ce que**
A représente H,
B représente Phe et
C représente Ile.

5. Composition pharmaceutique pour le traitement du syndrome de détresse respiratoire (RDS) chez les mammifères, **caractérisée par** la teneur en un polypeptide à activité surfactante pulmonaire selon l'une quelconque des revendications 1 à 4.

6. Composition pharmaceutique selon la revendication 5, **caractérisée en ce qu'**elle renferme au moins un autre polypeptide à activité de surfactant pulmonaire du groupe protéine surfactante pulmonaire SP-A et protéine surfactante pulmonaire SP-B.

7. Composition pharmaceutique selon la revendication 6, **caractérisée en ce qu'**elle renferme la protéine surfactante pulmonaire SP-B.

8. Composition pharmaceutique selon l'une quelconque des revendication 5 et 6, **caractérisée en ce qu'**elle renferme des phospholipides.

9. Composition pharmaceutique selon la revendication 8, **caractérisée en ce qu'**elle renferme de la dipalmitoyl-phosphatidyl-choline (DPPC), du palmitoyl-oléyl-phosphatidyl-glycérol (POPG) et/ou du phosphatidyl-glycérol (PG).

10. Composition pharmaceutique selon la revendication 8, **caractérisée en ce qu'**elle contient de l'acide palmitique et des électrolytes.

11. Composition pharmaceutique selon la revendication 10, **caractérisée en ce qu'**elle contient, en tant qu'électrolytes, des sels de calcium et/ou de sodium.
